# EUROPÄISCHE PATENTANMELDUNG

(11) **EP 2 706 054 A1**
(43) Veröffentlichungstag der Anmeldung: **12.03.2014**
(21) Anmeldenummer: 12183727.2
(22) Anmeldetag: 10.09.2012
(51) Int. Cl.: C07C 45/51

(54) **Verfahren zur Herstellung von Menthon aus Isopulegol**

(71) Anmelder: BASF SE, 67056 Ludwigshafen (DE)
(72) Erfinder: Die Erfindernennung liegt noch nicht vor
(74) Vertreter: Reitstötter - Kinzebach

(57) **Zusammenfassung**

Die vorliegende Erfindung betrifft ein Verfahren zur Darstellung von Menthon ausgehend von Isopulegol unter Verwendung spezieller homogener Katalysatoren.

## Beschreibung

Die vorliegende Erfindung betrifft ein Verfahren zur Darstellung von Menthon ausgehend von Isopulegol unter Verwendung spezieller homogener Katalysatoren.

### Hintergrund der Erfindung:

Menthon existiert in Form von zwei Stereoisomeren, Menthon **(**I**)** und Isomenthon **(II),** welche wiederum jeweils in Form zweier Enantiomere vorliegen. 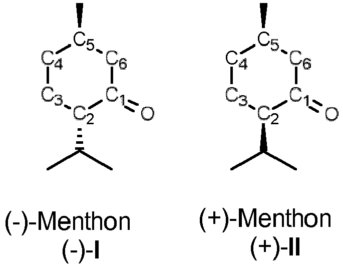 Beide Stereoisomere kommen in verschiedenen ätherischen Ölen vor, besonders in Ölen der Spezies *Mentha.* Menthone zeigen eine typische Minznote, während Isomenthone einen leichten Modergeruch aufweisen. Industrielle Menthone sind oft Mischungen der Isomere in verschiedenen Zusammensetzungen. Menthon und Isomenthon werden für synthetische Pfefferminzöle und -basen verwendet. (Quelle: K. Bauer, D. Garbe, H. Surburg, Common Fragrance and Flavour Compounds, 4th Edition, Wiley-VCH).

In der Literatur sind verschiedene Verfahren zur Herstellung von Menthon bekannt:
(-)-Menthon kann durch Destillation aus dem sogenannten *Dementholized Cornmint Oil,* einem Rückstand aus der Isolierung von (-)-Menthol aus dem ätherischen Öl der Acker- oder Kornminze (*mentha arvensis*)*,* gewonnen werden. Das *Dementholized Cornmint Oil* enthält 30-50% (-)-Menthon (Quelle: K. Bauer, D. Garbe, H. Surburg, Common Fragrance and Flavour Compounds, 4th Edition, Wiley-VCH). Genau wie im Falle des Menthols unterliegt aber auch Menthon aufgrund des natürlichen Ursprungs zyklischen Schwankungen, die zu Preisschwankungen führen. Zudem bedingt die Koppelproduktion, dass bei einer Verringerung des Anteils an (-)-Menthol aus natürlichen Quellen auch die Verfügbarkeit des dementholized cornmint oil abnimmt.

US 3,124,614 beschreibt die Synthese von Menthon durch Hydrierung von Thymol in Gegenwart von Pd/C-Katalysatoren. Auf diesem Weg ist jedoch nur ein Gemisch sämtlicher Stereoisomeren zugänglich, also eine Mischung von rac-Menthon und rac-Isomenthon.
(-)-Menthon kann auch durch Oxidation von (-)-Menthol hergestellt werden. Dafür kommen alle gängigen Methoden zur Oxidation von sekundären Alkoholen infrage z.B. Oxidationsmittel auf Basis giftiger Schwermetalle (z.B. Chromsäure oder Bichromat/Schwefelsäure) oder aber Sauerstoff bzw. Luft in Gegenwart eines Katalysators (z.B. N-Oxyl-Verbindungen, WO 2012008228).

WO 2005/085160 A1 beschreibt die Darstellung von Menthon durch Dehydrierung von Menthol in Gegenwart eines Katalysators in der Gasphase. Auf diese Weise erhält man eine Mischung aus Menthon und Isomenthon. Die Dehydrierung von Isopulegol zu Isopulegon ist ebenfalls erwähnt, nicht jedoch die Umsetzung von Isopulegon zu Menthon.

Eine ähnliche Gasphasendehydrierung von Menthol zu Menthon ist auch in DE 4236111 A1 beschrieben.

Allen diesen Publikationen ist gemein, dass sie für die Herstellung von Menthon von Menthol ausgehen. Atomökonomischer wäre es jedoch, Menthon ausgehend von Isopulegol herzustellen.

Die Direktsynthese von Menthon aus Isopulegol in der Gasphase an einem Kupferkontakt wird von W. Treibs und H. Schmidt in Berichte der Deutschen Chem. Gesellschaft 1927, 60B, 2335-41 beschrieben. Unter den dort beschriebenen Bedingungen fällt Thymol in erheblichen Mengen an (35%). Das Menthon wird als nicht näher beschriebenes Gemisch von (-)-Menthon und (+)-Isomenthon erhalten. Ni-Kontakte, welche z.B. auch für die Dehydrierung von Menthol zu Menthon verwendet werden können, führen hingegen zur Abspaltung von Wasser aus dem Isopulegol.

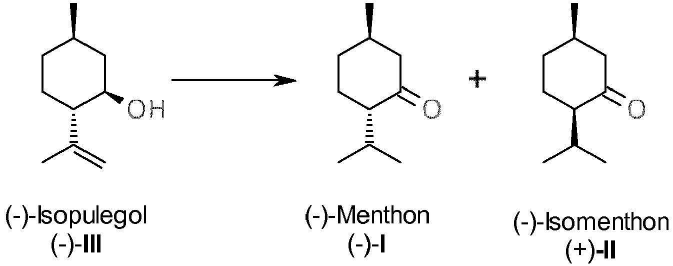

Aufgabe der vorliegenden Erfindung ist es daher, ein verbessertes Verfahren zur Herstellung von Menthon bereitzustellen.

### Kurzfassung der Erfindung:

Obige Aufgabe wurde erfindungsgemäß gelöst durch ein Verfahren, bei welchem unter milden Bedingungen das Menthon durch Hydrierung/Dehydrierung ausgehend von Isopulegol in hohen Ausbeuten erhalten wird.

Obige Aufgabe wurde erfindungsgemäß insbesondere gelöst durch das im Folgenden näher beschriebene Verfahren zur Herstellung von Menthon ausgehend von Isopulegol, welches man in einer Flüssigphase unter Verwendung eines homogen gelösten Katalysators, enthaltend mindestens ein Element ausgewählt aus den Gruppen 8, 9 und 10 des Periodensystems durchführt.

Das erfindungsgemäße Verfahren zeichnet sich gegenüber dem Stand der Technik durch folgende Vorteile aus:
• Atomökonomische Herstellung von Menthon aus Isopulegol durch Dehydrierung/Hydrierung, da alle Atome des Edukts im Produkt wieder zu finden sind
• Keine Racemisierung an Position C-5 .
• Deutlich verbesserte Selektivitäten (bis 90 %) gegenüber dem nächsten Stand der Technik (W. Treibs und H. Schmidt in *Berichte der Deutschen Chem. Gesellschaft,* 35 % Selektivität) durch Verwendung milderer Reaktionsbedingungen.
• Thymol wird nur in Spuren (< 0.05%) gebildet

### Figurenbeschreibung

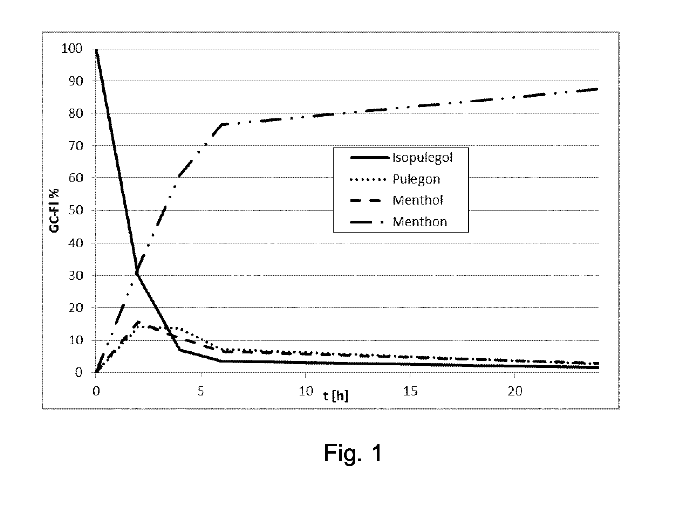

Figur 1 zeigt eine grafische Darstellung eines beispielhhaften erfindungsgemäßen Reaktionsverlaufs unter folgenden versuchsbedingungen: Einsatz von 20 g Isopulegol, 180°C Ölbadtemperatur, 0.3 Mol% [Ru(P*n*Oct₃)₄(H)₂]-Katalysator, Reaktion unter Inertbedingungen im Glaskolben mit Rückflußkühler; dargestellt ist die Abnaghme von Isopulegol under Bildung von Menthon und intermediärer Bildung von Menthol und Isopulegon ("Pulegon"); Analytik per GC

### Detaillierte Beschreibung der Erfindung:

### a) Besondere Ausführungsformen der Erfindung:

Die vorliegende Erfindung betrifft insbesondere folgende Ausführungsformen
1. Verfahren zur Herstellung von Menthon ausgehend von Isopulegol, **dadurch gekennzeichnet, dass** man eine Umlagerungsreaktion, insbesondere eine Dehydrierungs/Hydrierungs-Reaktion, d.h. Dehydrierung der OH-Gruppe am C1-Atom zur Ketogruppe, und eine Hydrierung der 1-Methylethenylgruppe am C2-Atom von Isopulegol, in der Flüssigphase unter Verwendung eines homogen gelösten Katalysators K, enthaltend wenigstens ein Metallatom aus der Gruppe 8, 9 oder 10, insbesondere 8 oder 9, des Periodensystems (IUPAC) durchführt.
2. Verfahren nach Ausführungsform 1, wobei der Katalysator K als Zentralatom M Ruthenium oder Iridium aufweist.
3. Verfahren nach einer der vorhergehenden Ausführungsformen, wobei der Katalysator K mindestens einen Phosphinliganden enthält.
4. Verfahren nach Ausführungsform 3, wobei der Katalysator K neben mindestens einem Phosphinliganden mindestens einen weiteren Liganden L aufweist, der ausgewählt ist unter CO, Hydrido, aliphatischen Olefinen, Cycloolefinen, carbocyclischen Aromaten, Heteroaromaten, Aldehyden, Ketonen, Halogeniden, C₁-C₄-Alkanoat, Methylsulfonat, Methylsulfat, Trifluormethylsulfat, Tosylat, Mesylat, Cyanid, Isocyanat, Cyanat, Thiocyanat, Hydroxid, C₁-C₄-Alkoxid, Cyclopentadienid, Pentamethylcyclopentadienid und Pentabenzylcyclopentadienid.
   Nichtlimitierende Beispiele
   für aliphatische Olefine sind C₂-C₄-Olefine, wie Ethylen, Propen, 1-Buten, 2-Buten, 2-Methyl-1-propen,
   für Cycloolefine sind Cyclopropen, Cyclobuten, Cyclobutadien, Cyclopentadien, Cyclhoxen, Cyclohexadien, Cycloocten, Cyclooctadien;
   für carbocyclische Aromaten sind Benzol, Naphthalin und Anthracen, 1-Isopropyl-4-Methylbenzol, Hexamtehylbenzol,
   für Heteroaromaten sind Pyridin, Lutidin, Picolin Pyrazin,
   für Aldehyde sind Formaldehyd, Acetaldehyd, Propionaldehyd, Butyraldehyd, iso-Butryaldehyd, Valeraldehyd, Isovaleraldhyd, Benzaldehyd,
   für Ketone sind Aceton, Menthon, für Halogenide sind F, Cl, Br, J,
   für C₁-C₄-Alkanoate sind Methanoat, Ethanoat, n-Propanoat und n-Butanoat.,
5. Verfahren nach einer der vorhergehenden Ausführungsformen, wobei der Katalysator K ausgewählt ist unter den Verbindungen:
   [Ru(PR₃)₄(H)₂] (R = Methyl, Ethyl, Butyl, Hexyl, Octyl, Phenyl, Tolyl, Mesityl),
   [Ru(PR₃)₃(H)₂(CO)] (R = Methyl, Ethyl, Butyl, Hexyl, Octyl, Phenyl, Tolyl, Mesityl), [Ru(PR₃)₃(H)(Cl)(CO)] (R = Methyl, Ethyl, Butyl, Hexyl, Octyl, Phenyl, Tolyl, Mesityl),
   [Ru(PR₃)₃(Cl)₂(CO)] (R = Methyl, Ethyl, Butyl, Hexyl, Octyl, Phenyl, Tolyl, Mesityl), [Ru(PR₃)₃(Cl)₂] (R = Methyl, Ethyl, Butyl, Hexyl, Octyl, Phenyl, Tolyl, Mesityl),
   [Ru(L2)₂(H)₂] (L2 = 1,2-Bisdicyclohexlyphosphinoethan, 1,2-Bisdiethylyphosphinoethan, 1,2-Bisdiphenylyphosphinoethan),
   [Ru(L2)(PR₃)₂(H)₂] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl, Tolyl, Mesityl),
   [Ru(L2)(PR₃)(CO)(H)₂] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl, Tolyl, Mesityl),
   [Ru(L2)(PR₃)(CO)(H)(Cl)] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl, Tolyl, Mesityl),
   [Ru(L3)(H)₂] (L3 = Triphos), und
   [Ru(L3)(CO)(H)₂] (L3 = Triphos),
   und
   [Ru(L3)(CO)(H)(C)] (L3 = Triphos)
6. Verfahren nach einem der vorhergehenden Ansprüche, wobei man den Katalysator K in einer Menge von 1 bis 5000 , 5 bis 2000 oder 10 bis 1000 ppm Gewichtsteilen, bezogen auf 1 Gewichtsteil des Isopulegols oder eines Gemischs aus einem Isopulegol mit wenigstens einem davon verschiedenen Alkohol, einsetzt.
7. Verfahren nach einer der vorhergehenden Ausführungsformen, wobei man ein Isomerengemisch des Isopulegols als Ausgangsverbindung einsetzt.
8. Verfahren nach einer der vorhergehenden Ausführungsformen, wobei man als Produkt ein Isomerengemisch des Menthons erhält.
9. Verfahren nach einer der vorhergehenden Ausführungsformen, wobei man die Reaktion ohne zusätzliches Lösungsmittel durchführt.
10. Verfahren nach einer der vorhergehenden Ausführungsformen, wobei man die Reaktion im Bereich von 100 bis 250 °C durchführt.
11. Reaktionsprodukt, erhältlich nach einem Verfahren gemäß einer der vorhergehenden Ausführungsformen.
12. Verwendung eines Reaktionsproduktes nach Ausführungsform 11 als Duftstoff oder Geschmacksstoff.
13. Zusammensetzung, enthaltend wenigstens ein Reaktionsprodukt nach Ausführungsform 11, wobei die Zusammensetzung ausgewählt ist unter Lebensmitteln, Süßigkeiten, Kaugummis, Getränken, Kosmetika, Zahnpasten, Mundspülungen, Shampoos, Toilettenartikeln, Lotionen, Hautpflege-Produkten, Medikamenten, Arzneimitteln.

### b) Edukte

Im erfindungsgemäßen Verfahren werden Isopulegole eingesetzt und auch Gemische verschiedener Isopulegole. Dabei werden als Isopulegol (1 R,2S,5R)-(-)-Isopulegol, (1R,2S,5R)-(-)-Isopulegol), (1S,2R,5R)-(+)-Isopulegol), (1S,2R,5S)-(+)-Isopulegol), (1S,2S,5R)-5-methyl-2-(1-methylethenyl)-Cyclohexanol oder (1R,2R,5R)-5-methyl-2-(1-methylethenyl)-Cyclohexanol bzw. Gemische dieser Isopulegole eingesetzt.

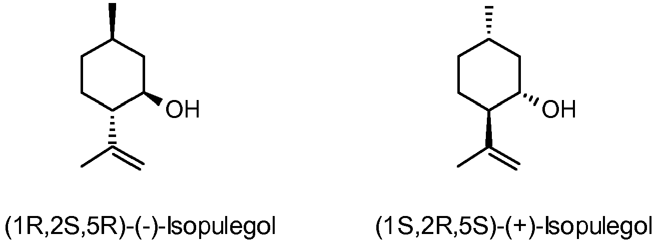

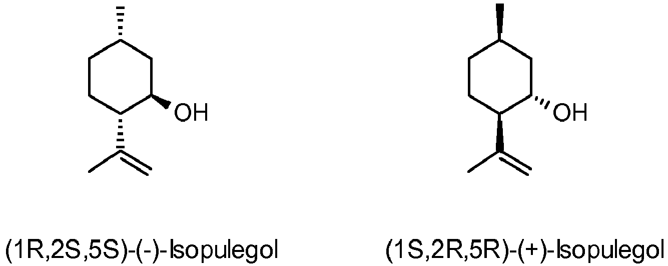

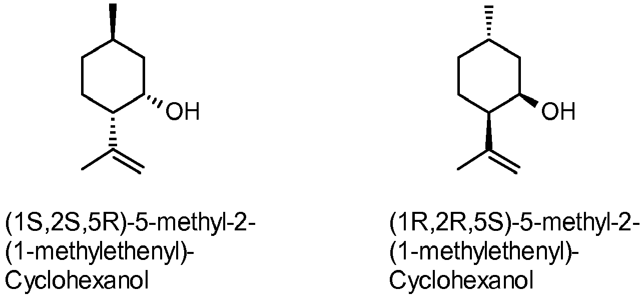

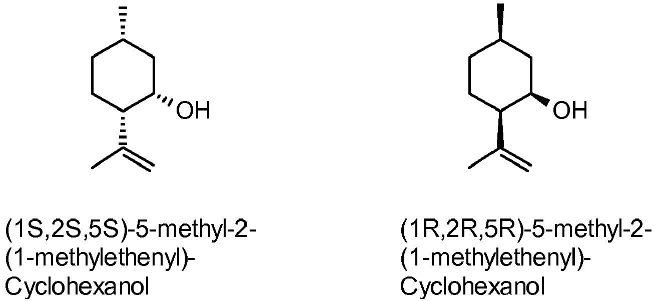

### c) Komplexkatalysatoren

Im erfindungsgemäßen Verfahren wird mindestens ein Komplexkatalysator, der mindestens ein Element ausgewählt aus den Gruppen 8, 9 und 10 des Periodensystems (Nomenklatur gemäß IUPAC) enthält, eingesetzt. Die Elemente der Gruppe 8, 9 und 10 des Periodensystems umfassen Eisen, Cobalt, Nickel, Ruthenium, Rhodium, Palladium, Osmium, Iridium und Platin. Bevorzugt sind Komplexkatalysatoren, die mindestens ein Element ausgewählt aus Ruthenium und Iridium enthalten. Der aktive Komplexkatalysator kann in seiner aktiven Form als auch in situ aus einer einfachen Metallvorstufe und einem geeignten Liganden im Reaktionsgemisch erzeugt werden. Als geeignete Metallvorstufen eigenen sich beispielsweise [Ru(p-cymene)Cl₂]₂, [Ru(benzol)Cl₂]ₙ, [Ru(CO)₂Cl₂]ₙ, [Ru(CO)₃Cl₂]₂ [Ru(COD)(allyl)], [RuCl₃*H₂O], [Ru(acteylacetonat)₃], [Ru(DMSO)₄Cl₂], [Ru(cyclopentadienyl)(CO)₂Cl], [Ru(cyclopentadienyl)(CO)₂H], [Ru(cyclopentadienyl)(CO)₂]₂, [Ru(pentamethylcyclopentadienyl)(CO)₂Cl], [Ru(pentamethylcylcopentadienyl)(CO)₂H], [Ru(pentamethylcyclopentadienyl)(CO)₂]₂, [Ru(indenyl)(CO)₂Cl], [Ru(indenyl)(CO)₂H], [Ru(indenyl)(CO)₂]₂, Ruthenocen, [Ru(COD)Cl₂]₂, [Ru(pentamethylcyclopentadienyl)(COD)CI], [Ru₃(CO)₁₂], [IrCl₃*H₂O], KIrCl₄, K₃IrCl₆, [Ir(COD)Cl]₂, [Ir(cycloocten)₂Cl]₂, [Ir(ethen)₂Cl]₂, [Ir(cyclopentadienyl)Cl₂]₂, [Ir(pentamethylcyclopentadienyl)Cl₂]₂ und [Ir(cylopentadienyl)(CO)₂], [Ir(pentamethylcyclopentadienyl)(CO)₂].

Als Ligand enthält der Katalysatorkomplex bevorzugt einen Phosphinliganden mit mindestens einem unverzweigten oder verzweigten, acyclischen oder cyclischen, aliphatischen oder arylischen Rest mit 1 bis 12 Kohlenstoffatomen enthalten, wobei einzelne Kohlenstoffatome auch durch >P- substituiert sein können. Im Sinne von verzweigten cyclischen aliphatischen Resten sind damit eingeschlossen auch Reste wie beispielsweise -CH₂-C₆H₁₁. Als geeignete Reste sind beispielsweise genannt Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, Cyclopentyl, Cyclohexyl, Cycloheptyl und Cyclooctyl, Methylcyclopentyl, Methylcyclohexyl, 1-(2-Methyl)-pentyl 1-(2-Ethyl)-hexyl, 1-(2-Propyl)heptyl und Norbonyl, Phenyl, Tolyl, Mesityl, Anisyl. Bevorzugt enthält der unverzweigte oder verzweigte, acyclische oder cyclische, aliphatische oder arylische Rest mindestens 1 sowie bevorzugt maximal 10 Kohlenstoffatome. Im Falle eines ausschließlich cyclischen Restes im oben genannten Sinne beträgt die Zahl der Kohlenstoffatome 3 bis 12 und bevorzugt mindestens 4 sowie bevorzugt maximal 8 Kohlenstoffatome. Bevorzugte Reste sind Ethyl, 1-Butyl, sec-Butyl, 1-Octyl und Cyclohexyl, Phenyl, Tolyl, Mesitly, Anisyl.

Die Phosphin-Gruppe kann einen, zwei oder drei der oben genannten unverzweigten oder verzweigten, acyclischen oder cyclischen, aliphatischen oder arylischen Reste enthalten. Diese können gleich oder verschieden sein. Bevorzugt enthält die Phosphin-Gruppe drei der oben genannten unverzweigten oder verzweigten, acyclischen oder cyclischen, aliphatischen Reste, wobei besonders bevorzugt alle drei Reste gleich sind. Bevorzugte Phosphine sind P(n-CₘH₂ₘ₊₁)₃ mit m gleich 1 bis 10, besonders bevorzugt Tri-n-butylphosphin, Tri-n-octylphosphin, Triphenylphosphin, Dipheynlphosphinoethan, Chiraphos, Triphos und 1,2-Bis(dicyclohexylphosphino)ethan.

Wie bereits weiter oben erwähnt, können in den genannten unverzweigten oder verzweigten, acyclischen oder cyclischen, aliphatischen Resten einzelne Kohlenstoffatome auch durch >P- substituiert sein. Somit sind auch mehrzähnige, beispielsweise zwei- oder dreizähnige, Phosphin-Liganden mit umfasst. Diese enthalten bevorzugt die Gruppierung >P-CH₂CH₂-P< beziehungsweise 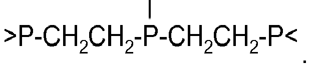

Enthält die Phosphin-Gruppe noch andere Reste als die oben genannten unverzweigten oder verzweigten, acyclischen oder cyclischen, aliphatischen Reste, so entsprechen diese im Allgemeinen denen, die ansonsten üblicherweise bei Phosphin-Liganden für metallorganische Komplexkatalysatoren eingesetzt werden. Als Beispiele seien genannt Phenyl, Tolyl und Xylyl.

Die metallorganische Komplexverbindung kann eine oder mehrere, beispielsweise zwei, drei oder vier, der oben genannten Phosphin-Gruppen mit mindestens einem unverzweigten oder verzweigten, acyclischen oder cyclischen, aliphatischen oder arylischen Rest enthalten.

Der Katalysatorkomplex kann noch weitere Liganden enthalten, als da wären Neutralliganden wie CO, Olefine, Cycloolefine, Diene, Cyclodiene, Aromaten, Aldehyde, Ketone sowie anionische Liganden wie Fluorid, Chlorid, Bromid, lodid, Hydrid, Formiat, Acetat, Propionat, Butyrat, Methylsulfonat, Methylsulfat, Tifluormethylsulfat, Tosylat, Mesylat, Cyanid, Isocyanat, Thiocyanat, Hydroxid, Alkoxid, Cylopentadienid, Pentamethylcyclopentadienid und Pentabenzylcyclopentadienid.

Als bevorzugte Katalysatoren werden Katalysatoren des Typs [Ru(PR₃)₄(H)₂] (R = Methyl, Ethyl, Butyl, Hexyl, Octyl, Phenyl. Tolyl, Mesityl), [Ru(PR₃)₃(H)₂(CO)] (R = Methyl, Ethyl, Butyl, Hexyl, Octyl, Phenyl. Tolyl, Mesityl), [Ru(PR₃)₃(H)(Cl)(CO)] (R = Methyl, Ethyl, Butyl, Hexyl, Octyl, Phenyl. Tolyl, Mesityl), [Ru(PR₃)₃(Cl)₂(CO)] (R = Methyl, Ethyl, Butyl, Hexyl, Octyl, Phenyl. Tolyl, Mesityl), [Ru(PR₃)₃(Cl)₂] (R = Methyl, Ethyl, Butyl, Hexyl, Octyl, Phenyl. Tolyl, Mesityl), [Ru(L2)₂(H)₂] (L2 = 1,2-Bisdicyclohexlyphosphinoethan, 1,2-Bisdiethylyphosphinoethan, 1,2-Bisdiphenylyphosphinoethan), [Ru(L2)(PR₃)₂(H)₂] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl. Tolyl, Mesityl),
[Ru(L2)(PR₃)(CO)(H)₂] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl. Tolyl, Mesityl),
[Ru(L2)(PR₃)(CO)(H)(Cl)] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl. Tolyl, Mesityl),
[Ru(L3)(H)₂] (L3 = Triphos),
[Ru(L3)(CO)(H)₂] (L3 = Triphos),
[Ru(L3)(CO)(H)(C)] (L3 = Triphos) eingesetzt.

Es kann auch vorteilhaft sein, dem Reaktionsgemisch eine Base zuzusetzen. Als geeignete Basen seien LiOH, NaOH, KOH, LiH, NaH, KH, Ca(OH)₂, CaH₂, LiAlH₄, NaBH₄, LiBH₄, Na₂CO₃, NaHCO₃, Li₂CO₃, LiHCO₃, K₂CO₃, KHCO₃, K₃PO₄, Na₃PO₄, BuLi, MeLi, PhLi, tBuLi, LiOMe, LiOEt, LiOPr, LiO*i*Pr, LiOBu, LiO*i*Bu, LiOPent, LiO*i-*Pent, LiOHex, LiOHept, LiOOct, LiOBenz, LiOPh, KOMe, KOEt, KOPr, KO*i*Pr, KOBu, KO*i*Bu, KOPent, KO*i*Pent, KOHex, KOHept, KOOct, KOBenz, KOPh, NaOMe, NaOEt, NaOPr, NaO*i*Pr, NaOBu, NaO*i*Bu, NaOPent, NaO*i*Pent, NaOHex, NaOHept, NaOOct, NaOBenz, NaOPh, KN(SiMe₃)₂, LiN(SiMe₃)₃, NaN(SiMe₃)₃, NH₃, RNH₂ (mit R₁ = unsubstituiertes oder zumindest monosubstituiertes C₁-C₁₀-Alkyl, H, (-C₁-C₄-Alkyl-P(Phenyl)₂), C₃-C₁₀-Cycloalkyl, C₃-C₁₀-Heterocyclyl sind enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S), R₁R₂NH (mit R₁, R₂ unabhängig voneinander unsubstituiertes oder zumindest monosubstituiertes C₁-C₁₀-Alkyl, H, (-C₁-C₄-Alkyl-P(Phenyl)₂), C₃-C₁₀-Cycloalkyl, C₃-C₁₀-Heterocyclyl sind enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S), R₁R₂R₃N (mit R₁, R₂, R₃ unabhängig voneinander unsubstituiertes oder zumindest monosubstituiertes C₁-C₁₀-Alkyl, H, (-C₁-C₄-Alkyl-P(Phenyl)₂), C₃-C₁₀-Cycloalkyl, C₃-C₁₀-Heterocyclyl sind enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S) genannt.

### d) Umlagerung

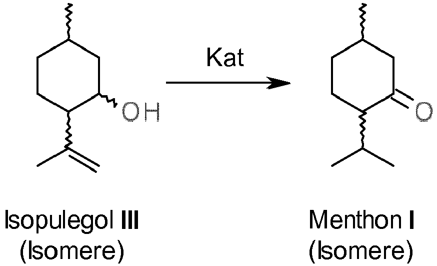

Die Bildung von Menthon aus Isopulegol erfolgt durch die Dehydrierung der Hydroxygruppe zu einer Carbonylfunktion unter Hydrierung der C-C-Doppelbindung des Substrates. Dabei kann ein reines Isomer des Isopulegols wie auch Isomerengemisch eingesetzt werden. Das bei der Reaktion erhaltenen Menthon ist isomerenrein oder es wird auch als Isomerengemisch erhalten.

Bevorzugt führt man die Reaktion mit (-)-Isopulegol durch, wobei sich als Hauptprodukt (-)-Menthon und (+)-Isomenthon bilden.

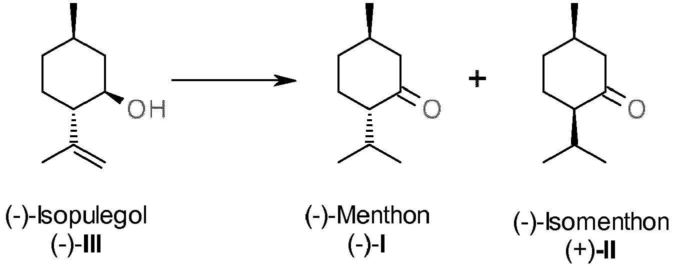

Unter "homogen-katalysiert" wird im Rahmen der vorliegenden Erfindung verstanden, dass der katalytisch aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst im flüssigen Reaktionsmedium vor (100 %), jeweils bezogen auf die Gesamtmenge im flüssigen Reaktionsmedium.

Die Menge der Metallkomponente des Katalysators, bevorzugt Ruthenium, beträgt im Allgemeinen 0,1 bis 5000 Gewichts-ppm, jeweils bezogen auf das gesamte flüssige Reaktionsgemisch im Reaktionsraum.

Die Umsetzung erfolgt in der Flüssigphase im Allgemeinen bei einer Temperatur von 20 bis 250°C. Bevorzugt wird das erfindungsgemäße Verfahren bei Temperaturen im Bereich von 100 °C bis 200 °C, besonders bevorzugt im Bereich von 100 bis 180°C durchgeführt.

Die Reaktion wird im Allgemeinen bei einem Gesamtdruck von 0.1 bis 20 MPa absolut, der sowohl der Eigendruck des Lösungsmittels bzw. des Substrates bei der Reaktionstemperatur als auch der Druck eines Gases wie Stickstoff, Argon oder Wasserstoff sein kann, durchgeführt werden. Bevorzugt wird das erfindungsgemäße Verfahren bei einem Gesamtdruck bis 10 MPa absolut, insbesondere bevorzugt bei einem Gesamtdruck bis 1 MPa absolut durchgeführt.

Beim erfindungsgemäßen Verfahren kann die Umsetzung sowohl mit einem zusätzlichen Lösungsmittel als auch ohne Lösungsmittelzusatz durchgeführt werden. Als Lösungsmittel eigenen sich beispielsweise aliphatische und aromatische Kohlenwasserstoffe, aliphatische und aromatische Ether, Cyclische Ether oder Ester. Genannt seien hier, aber nicht einschränkend, beispielsweise Lösungsmittel wie Pentan, Hexan, Heptan, Oktan, Nonan, Dekan, Benzol, Toluol, Xylole, Mesitylen, Anisol, Dibutylether, Diphenylether, Dimethoxyethan, Tetrahydrofuran, Methyltetrahydrofuran, Dioxan, Ethylacetat, Butylacetat oder Butylbutyrat genannt. Wird die Reaktion ohne zusätzliches Lösungsmittel durchgeführt, so muss auch das Produkt nicht von diesem abgetrennt werden, was die Aufarbeitung vereinfacht. Bei der Ausführungsform ohne Lösungsmittel erfolgt die Umsetzung in den Reaktanden bzw. dem bei der Reaktion entstehenden Produktes.

Für die Umsetzung in der Flüssigphase leitet man mindestens ein Isopulegol und ggf. das Lösungsmittel, den Metallkatalysator oder eine geeignete Metallvorstufe und die Liganden ggf. zusätzlich eine Base, in einen Reaktionsraum. Die Reaktion kann in den üblichen, dem Fachmann bekannten Vorrichtungen bzw. Reaktoren für FlüssigGas-Reaktionen, bei denen der Katalysator homogen gelöst in der Flüssigphase vorliegt, durchgeführt werden. Für das erfindungsgemäße Verfahren können prinzipiell alle Reaktoren eingesetzt werden, welche für Gas-flüssig-Reaktionen unter der gegebenen Temperatur und dem gegebenen Drucks grundsätzlich geeignet sind. Geeignete Standardreaktoren für Gas-flüssig und für flüssig-flüssig Reaktionssysteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren. Die Zuleitung von Isopulegol und ggf. das Lösungsmittel, den Metallkatalysator oder eine geeignete Metallvorstufe und die Liganden ggf. zusätzlich eine Base, kann dabei simultan oder getrennt voneinander erfolgen. Die Reaktion kann dabei diskontinuierlich in Batchfahrweise oder kontinuierlich, semikontinuierlich mit Rückführung oder ohne Rückführung durchgeführt werden. Die mittlere Verweilzeit im Reaktionsraum beträgt im Allgemeinen 15 Minuten bis 100 Stunden.

### e) Aufreinigung

Nach der Reaktion wird das Produkt von nicht umgesetzten Edukten und ggf. dem Lösungsmittel vorzugsweise durch Destillation abgetrennt. Der Katalysator verbleibt mit den Hochsiedern im Sumpf der Destillation zurück und kann wiederverwendet werden. Nicht umgesetzter Eduktalkohol kann ebenfalls wieder in die Reaktion zurückgeführt werden. Die thermische Abtrennung des Alkoholes sowie des Esters erfolgt nach den dem Fachmann bekannten Verfahren des Standes der Technik, bevorzugt in einem Verdampfer oder in einer Destillationseinheit, umfassend Verdampfer und Kolonne(n), die üblicherweise mehrere Böden, eine Packung oder Füllkörper aufweist. Die Abtrennung des Produktes kann auch durch Kristallisation, Extraktion oder Absorption erfolgen, wobei die destillative Aufarbeitung bevorzugt ist.

Nicht umgesetztes Edukt sowie der Metallkatalysator werden bevorzugt für die Reaktion wiederverwendet, um ein möglichst ökonomisches Verfahren zu ermöglichen.

Die Erfindung wird durch die nachfolgenden Beispiele verdeutlicht, ohne sie darauf zu beschränken:

### Experimenteller Teil:

### Allgemeine Angaben:

Die gaschromatographischen Bestimmungen werden wie folgt durchgeführt: Die GC-Bestimmung der Ausbeuten wurden auf einem Agilent-Gerät mit einer VF-23ms Säule (60 m, 0.25 mm, 0.25 µm), Helium als Trägergas und einem Flammionisationsdetektor durchgeführt. Die Injektortemperatur betrug 250°C, die Säulentemperatur wurde währende der Messung von 50°C mit einer Aufheizrate von 3°C/min auf 150°C und dann mit 20°C/min auf 260°C erhöht.

### In den folgenden Beispielen gelten folgende Definitionen:

"Inertbedingungen": Die Arbeiten wurden unter Ausschluss von Luft bzw. Sauerstoff durchgeführt. Die Einwaage des Eduktes, Lösungsmittels und des Katalysators erfolgte in einer Handschuhbox, welche mit Reinststickstoff betrieben wird. Die Arbeiten außerhalb der Handschuhbox erfolgten unter Anwendung von Standart-Schlenk Techniken und Argon als Inertgas.

### "Normaldruck": Umgebungsdruck, etwa 1 atm

Ausbeuten: Bei der Umlagerung des Isopulegols zum Menthon treten Menthol sowie Isopulegon als Intermediate auf. Durch eine längerer Reaktionszeit bzw. Recycling dieser Intermediate können diese weiter zum Menthon umgesetzt werden, stellen somit also keinen Verlust an Isopulegol bezgl. der Synthese von Menthon dar (siehe exemplarischen Reaktionverlauf im abgebildeten Graphen gemäß Fig. 1 bzgl. dieser Komponenten). Diese Nebenkomponenten sind bei den Ausbeuten und Selektivitäten deshalb explizit aufgeführt.

### Herstellungsbeispiel 1:

Unter Inertbedingungen werden 202 mg [Ru(P*n*Oct₃)₄(H)₂], 1.8 g Isopulegol und 10 mL o-Xylol (wasserfrei) in einer Glovebox in einen 50 mL Zweihalskolben eingewogen. Das Reaktionsgemisch wird dann bei Normaldruck unter Rückflusskühlung bei 133°C Ölbadtemperatur 12 Stunden gerührt. Nach der Reaktion wird der Umsatz und die Ausbeute am Menthon (Summe der Isomere) durch Gaschromatographie (FI%) bestimmt. Der Umsatz am Isopulegol beträgt 60.5 % bei einer Selektivität bezüglich Menthon (Isomerengemisch aus 66.2 % (-)-Menthon, 33.8 % (+)-Isomenthon) von 47.3 %. Selektivität bzgl. der Nebenkomponenten: Menthol 31.6 %, Isopulegon 14.9 %, Gesamtselektivität (Menthon + Menthol + Isopulegon) 93.8 %.

### Herstellungsbeispiel 2:

Unter Inertbedingungen werden 116 mg [Ru(P*n*Bu₃)₄(H)₂], 54 mg 1,2-Bis(dicyclohexylphosphino)ethan, 1.8 g Isopulegol und 10 mL o-Xylol (wasserfrei) in einer Glovebox in einen 50 mL Zweihalskolben eingewogen. Das Reaktionsgemisch wird dann bei Normaldruck unter Rückflusskühlung bei 133°C Ölbadtemperatur 12 Stunden gerührt. Nach der Reaktion wird der Umsatz und die Ausbeute am Menthon (Summe der Isomere) durch Gaschromatographie (FI%) bestimmt. Der Umsatz am Isopulegol beträgt 62.9 % bei einer Selektivität bezüglich Menthon (Isomerengemisch aus 61.7 % (-)-Menthon, 38.3 % (+)-Isomenthon) von 31,9 %. Selektivität bzgl. der Nebenkomponenten: Menthol 27.7 %, Isopulegon 15.5 %, Gesamtselektivität (Menthon + Menthol + Isopulegon) 75.1 %.

### Herstellungsbeispiel 3:

Unter Inertbedingungen werden 116 mg [Ru(P*n*Et₃)₄(H)₂], 1.8 g Isopulegol und 10 mL o-Xylol (wasserfrei) in einer Glovebox in einen 50 mL Zweihalskolben eingewogen. Das Reaktionsgemisch wird dann bei Normaldruck unter Rückflusskühlung bei 133°C Ölbadtemperatur 12 Stunden gerührt. Nach der Reaktion wird der Umsatz und die Ausbeute am Menthon (Summe der Isomere) durch Gaschromatographie (FI%) bestimmt. Der Umsatz am Isopulegol beträgt 64.2 % bei einer Selektivität bezüglich Menthon (Isomerengemisch aus 70.0 % (-)-Menthon, 33.0 % (+)-Isomenthon) von 45.2 %. Selektivität bzgl. der Nebenkomponenten: Menthol 30.8 %, Isopulegon 20.2 %, Gesamtselektivität (Menthon + Menthol + Isopulegon) 96.2 %.

### Herstellungsbeispiel 4:

Unter Inertbedingungen werden 404 mg [Ru(P*n*Oct₃)₄(H)₂], 3.6 g Isopulegol und 10 mL o-Xylol (wasserfrei) in einer Glovebox in einen 50 mL Glasautoklaven eingewogen. Das Reaktionsgemisch wird dann bei unter Eigendruck (0.5 bar Überdruck) bei 130°C Ölbadtemperatur 12 Stunden gerührt. Nach der Reaktion wird der Umsatz und die Ausbeute am Menthon (Summe der Isomere) durch Gaschromatographie (FI%) bestimmt. Der Umsatz am Isopulegol beträgt 64.5 % bei einer Selektivität bezüglich Menthon (Isomerengemisch aus 65.8 % (-)-Menthon, 34.2 % (+)-Isomenthon) von 46.3 %. Selektivität bzgl. der Nebenkomponenten: Menthol 30.2 %, Isopulegon 14.4 %, Gesamtselektivität (Menthon + Menthol + Isopulegon) 90.9 %.

### Herstellungsbeispiel 5:

Unter Inertbedingungen werden 404 mg [Ru(P*n*Oct₃)₄(H)₂], 3.6 g Isopulegol und 20 mL o-Xylol (wasserfrei) in einer Glovebox in einen 50 mL Glasautoklaven eingewogen. Das Reaktionsgemisch wird dann bei unter Eigendruck bei 150°C Ölbadtemperatur 12 Stunden gerührt. Nach der Reaktion wird der Umsatz und die Ausbeute am Menthon (Summe der Isomere) durch Gaschromatographie (FI%) bestimmt. Der Umsatz am Isopulegol beträgt 92.4 % bei einer Selektivität bezüglich Menthon (Isomerengemisch aus 65.6 % (-)-Menthon, 34.4 % (+)-Isomenthon) von 51.1 %. Selektivität bzgl. der Nebenkomponenten: Menthol 15.4 %, Isopulegon 10.9 %, Gesamtselektivität (Menthon + Menthol + Isopulegon) 77.4 %.

### Herstellungsbeispiel 6:

Unter Inertbedingungen werden 460 mg [Ru(P*n*Oct₃)₄(H)₂], 8.6 g Isopulegol und 20 mL o-Xylol (wasserfrei) in einer Glovebox in einen 50 mL Glasautoklaven eingewogen. Das Reaktionsgemisch wird dann bei unter Eigendruck bei 170°C Ölbadtemperatur 12 Stunden gerührt. Nach der Reaktion wird der Umsatz und die Ausbeute am Menthon (Summe der Isomere) durch Gaschromtaographie (FI%) bestimmt. Der Umsatz am Isopulegol beträgt 98,2 % bei einer Selektivität bezüglich Menthon (Isomerengemisch aus 65.4 % (-)-Menthon, 34.6 % (+)-Isomenthon) von 89,6 %. Selektivität bzgl. der Nebenkomponenten: Menthol 3.6 %, Isopulegon 0.3 %, Gesamtselektivität (Menthon + Menthol + Isopulegon) 93.5 %.

### Herstellungsbeispiel 7:

Unter Inertbedingungen werden 300 mg [Ru(P*n*Oct₃)₄(H)₂] und 21,0 g Isopulegol in einer Glovebox in einen 50 mL Glasautoklaven eingewogen. Das Reaktionsgemisch wird dann bei unter Eigendruck bei 170°C Ölbadtemperatur 100 Stunden gerührt. Nach der Reaktion wird der Umsatz und die Ausbeute am Menthon (Summe der Isomere) durch Gaschromtaographie (FI%) bestimmt. Der Umsatz am Isopulegol beträgt 99,3 % bei einer Selektivität bezüglich Menthon Isomerengemisch aus 64.3 % (-)-Menthon, 35.7 % (+)-Isomenthon) von 86,4%. Selektivität bzgl. der Nebenkomponenten: Menthol 1.4 %, Isopulegon 4.9 %, Gesamtselektivität (Menthon + Menthol + Isopulegon) 92.7 %.

### Herstellungsbeispiel 8:

Unter Inertbedingungen werden 610 mg [Ru(P*n*Oct₃)₄(H)₂] und 20,15 g Isopulegol in einer Glovebox in einen 100 mL Glaskolben eingewogen. Das Reaktionsgemisch wird dann bei unter Rückfluß bei 170°C Ölbadtemperatur 24 Stunden gerührt. Nach der Reaktion wird der Umsatz und die Ausbeute am Menthon (Summe der Isomere) durch Gaschromtaographie (FI%) bestimmt. Der Umsatz am Isopulegol beträgt 97.5 % bei einer Selektivität bezüglich Menthon Isomerengemisch aus 63.1 % (-)-Menthon, 36.9 % (+)-Isomenthon) von 84.0 %. Selektivität bzgl. der Nebenkomponenten: Menthol 5.1 %, Isopulegon 4.5 %, Gesamtselektivität (Menthon + Menthol + Isopulegon) 93.7 %.

### Herstellungsbeispiel 9:

Unter Inertbedingungen werden 610 mg [Ru(P*n*Oct₃)₄(H)₂] und 20,7 g Isopulegol in einer Glovebox in einen 100 mL Glaskolben eingewogen. Das Reaktionsgemisch wird dann bei unter Rückfluß bei 180°C Ölbadtemperatur 24 Stunden gerührt. Nach der Reaktion wird der Umsatz und die Ausbeute am Menthon (Summe der Isomere) durch Gaschromtaographie (FI%) bestimmt. Der Umsatz am Isopulegol beträgt 98.5 % bei einer Selektivität bezüglich Menthon Isomerengemisch aus 63.0 % (-)-Menthon, 37.0 % (+)-Isomenthon) von 88.7 %. Selektivität bzgl. der Nebenkomponenten: Menthol 2.9 %, Isopulegon 2.7 %, Gesamtselektivität (Menthon + Menthol + Isopulegon) 94.3 %.

Auf die Offenbarung der hierin zitierten Druckschriften wird ausdrücklich bezug genommen.

## Patentansprüche

1. Verfahren zur Herstellung von Menthon ausgehend von Isopulegol, **dadurch gekennzeichnet, dass** man eine Dehydrierungs/Hydrierungs-Reaktion in der Flüssigphase unter Verwendung eines homogen gelösten Katalysators K enthaltend wenigstens ein Metallatom aus der Gruppe 8, 9 oder 10 des Periodensystems (IUPAC) durchführt.

2. Verfahren nach Anspruch 1, wobei der Katalysator K als Zentralatom M Ruthenium oder Iridium aufweist.

3. Verfahren nach einem der vorhergehenden Ansprüche, wobei der Katalysator K mindestens einen Phosphinliganden enthält.

4. Verfahren nach Anspruch 3, wobei der Katalysator K neben mindestens einem Phosphinliganden mindestens einen weiteren Liganden L aufweist, der ausgewählt ist unter CO, Hydrido, aliphatischen Olefinen, Cycloolefinen, carbocyclischen Aromaten, Heteroaromaten, Aldehyden, Ketonen, Halogeniden, C₁-C₄-Alkanoat, Methylsulfonat, Methylsulfat, Trifluormethylsulfat, Tosylat, Mesylat, Cyanid, Isocyanat, Cyanat, Thiocyanat, Hydroxid, C₁-C₄-Alkoxid, Cyclopentadienid, Pentamethylcyclopentadienid und Pentabenzylcyclopentadienid.

5. Verfahren nach einem der vorhergehenden Ansprüche, wobei der Katalysator K ausgewählt ist unter den Verbindungen:
[Ru(PR₃)₄(H)₂] (R = Methyl, Ethyl, Butyl, Hexyl, Octyl, Phenyl. Tolyl, Mesityl),
[Ru(PR₃)₃(H)₂(CO)] (R = Methyl, Ethyl, Butyl, Hexyl, Octyl, Phenyl. Tolyl, Mesityl),
[Ru(PR₃)₃(H)(Cl)(CO)] (R = Methyl, Ethyl, Butyl, Hexyl, Octyl, Phenyl. Tolyl, Mesityl),
[Ru(PR₃)₃(Cl)₂(CO)] (R = Methyl, Ethyl, Butyl, Hexyl, Octyl, Phenyl. Tolyl, Mesityl), [Ru(PR₃)₃(Cl)₂] (R = Methyl, Ethyl, Butyl, Hexyl, Octyl, Phenyl. Tolyl, Mesityl),
[Ru(L2)₂(H)₂] (L2 = 1,2-Bisdicyclohexlyphosphinoethan, 1,2-Bisdiethylyphosphinoethan, 1,2-Bisdiphenylyphosphinoethan),
[Ru(L2)(PR₃)₂(H)₂] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl. Tolyl, Mesityl),
[Ru(L2)(PR₃)(CO)(H)₂] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl. Tolyl, Mesityl),
[Ru(L2)(PR₃)(CO)(H)(Cl)] (L2 = 1,2-Bisdicyclohexlyphosphinoethan, 1,2-Bisdiethylyphosphinoethan, 1,2-Bisdiphenylyphosphinoethan; R = Methyl, Ethyl, Butyl, Hexyl, Octyl, Phenyl. Tolyl, Mesityl),
[Ru(L3)(H)₂] (L3 = Triphos), und
[Ru(L3)(CO)(H)₂] (L3 = Triphos),
und
[Ru(L3)(CO)(H)(C)] (L3 = Triphos)

6. Verfahren nach einem der vorhergehenden Ansprüche, wobei man den Katalysator K in einer Menge von 1 bis 5000 ppm Gewichtsteilen, bezogen auf 1 Gewichtsteil des Isopulegols oder eines Gemischs aus einem Isopulegol mit wenigstens einem davon verschiedenen Alkohol, einsetzt.

7. Verfahren nach einem der vorhergehenden Ansprüche, wobei man ein Isomerengemisch des Isopulegols als Ausgangsverbindung einsetzt.

8. Verfahren nach einem der vorhergehenden Ansprüche, wobei man als Produkt ein Isomerengemisch des Menthons erhält.

9. Verfahren nach einem der vorhergehenden Ansprüche, wobei man die Reaktion ohne zusätzliches Lösungsmittel durchführt.

10. Verfahren nach einem der vorhergehenden Ansprüche, wobei man die Reaktion im Bereich von 100 bis 250 °C durchführt.

11. Reaktionsprodukt, erhältlich nach einem Verfahren gemäß einem der vorhergehenden Ansprüche.

12. Verwendung eines Reaktionsproduktes nach Anspruch 11 als Duftstoff oder Geschmacksstoff.

13. Zusammensetzung, enthaltend wenigstens ein Reaktionsprodukt nach Anspruch 11, wobei die Zusammensetzung ausgewählt ist unter Lebensmitteln, Süßigkeiten, Kaugummis, Getränken, Kosmetika, Zahnpasten, Mundspülungen, Shampoos, Toilettenartikeln, Lotionen, Hautpflege-Produkten, Medikamenten, Arzneimitteln.
